# EUROPEAN PATENT APPLICATION

(11) **EP 4 668 285 A1**
(43) Date of publication of application: **24.12.2025**
(21) Application number: 24216487.9
(22) Date of filing: 29.11.2024
(51) Int. Cl.: G16H 30/20, G16H 40/20

(54) **METHOD FOR PROVIDING MEDICAL IMAGING WORKFLOW SUPPORT DATA**

(71) Applicant: Siemens Healthineers AG, 91301 Forchheim (DE)
(72) Inventor: KATZMANN, Alexander, 90765 Fürth (DE); TAUBMANN, Oliver, 91365 Weilersbach (DE); SÜHLING, Michael, 91052 Erlangen (DE)
(74) Representative: Siemens Healthineers Patent Attorneys

(57) **Abstract**

In one aspect the invention relates to a computer-implemented method for providing medical imaging workflow support data, the method comprising:
- receiving (S1) natural language data (U), the natural language data (U) comprising clinical information,
- generating (S2) structured information (N) by applying a large language model (L) to the natural language data (U), the structured information (N) comprising the clinical information in a structured format of at least one input node (31, 32) of an inference graph (G),
- calculating (S3) the medical imaging workflow support data by applying at least one rule of the inference graph (G) to the structured information (N), and
- providing (S4) the medical imaging workflow support data.

## Description

In one aspect the invention relates to a computer-implemented method for providing medical imaging workflow support data. In other aspects, the invention relates to a data processing system, a medical imaging system, a computer program product and a computer-readable storage medium.

In clinical IT systems, patient information is typically stored fragmented, widely distributed over various IT systems, and often present in the form of unstructured reports only. As was shown by recent studies, this may result in a significant loss of vital information, directly impacting timely and accurate patient treatment, and therefore poses a significant obstacle toward efficient workflow automation and accurate patient care.

At the same time, scanning complexity is rising, primarily due to technological advancements, a shift from the traditional one-size-fits-all model to a personalized medicine approach, and, not least, increasing legal requirements. This situation is exacerbated by a growing staff shortage in healthcare settings, resulting in a widening gap between workload on the one hand and available personnel on the other.

Under these circumstances, missing information often translates into delayed or disrupted care for a substantial portion of patients, which may significantly increase risks and is compromising outcomes. While standardized vocabularies and protocols (e.g. ICD-10, FHIR, ...) exist, their practical application within diverse clinical IT landscapes is far from omnipresent, in particular due to data silos, vendor-specific solutions, and, if using non-standardized formats, the inherent ambiguity of natural language in medical documentation.

Therefore, crucial details are often lost in translation from unstructured reports to structured formats, if at all, impacting downstream processes and increasing the risk of delayed or disrupted treatments. Furthermore, clinicians must manually decipher complex reports, map findings, and adapt protocols, diverting valuable time and effort from patient care. Moreover, with increasing complexity of image acquisition techniques, the process of mapping clinical questions into acquisition protocols, as well as their manual refinement to achieve patient-tailored imaging further increases workload and cognitive burden, and results in a reduced focus on the patient.

Due to the imperfection of the available information, decisions are often intuitive and/or based on substantial experience of the treating personnel. This process may lack transparency, explainability and/or traceability, making it challenging to understand rationale and update workflows in response to evolving clinical practices and technological advancements.

In many cases, scanners typically only receive abstract descriptions of the procedure to be performed via the DICOM Modality Worklist (DMWL), which in many cases is insufficient for advanced workflow automation. For example, on-the-fly adaptation of protocols may be limited to user-provided answers to predefined questions based, for example, on decision trees, and/or a manual setting of parameters and steps to be performed. This process relies fully on the expertise of the scanner-operating technologists and often requires time-consuming additional communication with radiologists and referring physicians in case of missing or unclear information.

Further, general scan and postprocessing Standard Operating Procedures (SOPs) are thus still largely modeled as individual specialized protocols, which may in practice lead to a large and complex set of protocols that can be difficult to maintain and requires expert knowledge to properly set up via the corresponding configuration Uls. This is usually done by lead technologists based on requirements posed by the radiologists. If they need support in this process, both in terms of what the best options are for a particular scanner and how to perform the configuration correctly, they are often limited to browsing manuals or asking application specialists who may not be aware of every relevant aspect due to the abundance of options.

The creation and maintenance of scan protocols at the scanner is a costly and time-consuming process. This is primarily a result of the numerous specialized protocols required to meet clinical demands on the one hand and the increasing capabilities, and thus configuration parameters of the scanners on the other. Different scanners with varying capabilities and software versions further complicate the representation of scan protocols. Moreover, on-site, scan protocols are typically adapted and thus specific to each clinic, making it impossible to provide all relevant configurations ex-factory. Therefore, each site maintains its own protocols, e.g., in a protocol book, and ensures they are accurately reflected on each scanner.

For example, CT scan protocols can be exported and imported from and to a scanner as XML files using a proprietary internal schema. However, this schema is not explicitly defined and is only understood through its implementation. Modifying protocol files requires extensive knowledge and understanding due to their large and complex nature. Furthermore, each new scanner software version may require a separate definition and implementation for transferring earlier protocols or protocol versions. Some complementary aspects, such as the clinical decision trees associated with the protocols, may be defined in separate files with similarly specified formats.

Given an abstract high-level protocol specification for a particular patient, scanner-specific protocols currently would have to be selected and configured separately for each scanner, as acquisition and post-processing parameters differ with every scanner and version. Therefore, handling and maintaining scan protocols is often challenging.

The typical process of creating and maintaining scan protocols is manual and relies heavily on the expertise of protocol administrators. This labor-intensive task requires a thorough understanding of the protocols to ensure their accurate implementation and ongoing maintenance, both in a central document (protocol book) and directly on each of the scanners using a user interface for exam protocol definition. This manual process is costly and requires extensive knowledge of the different scanners, software versions, and protocols used at each site. This becomes even more crucial for photon counting computed tomography, which offer many additional capabilities, such as always-on spectral acquisition and ultra-high resolution reconstructions.

Prior art related to this technical field is disclosed, for example, in EP 4 462 445 A1.

The underlying technical problem of the invention is to provide an alternative and/or an improvement to already known solutions for medical imaging workflow support. This problem is solved by the subject matter of the independent claims. The dependent claims are related to further aspects of the invention. Independent of the grammatical term usage, individuals with male, female or other gender identities are included within the term.

In one aspect the invention relates to a computer-implemented method for providing medical imaging workflow support data, the method comprising:
- receiving natural language data, the natural language data comprising clinical information,
- generating structured information by applying a large language model to the natural language data, the structured information comprising the clinical information in a structured format of at least one input node of an inference graph,
- calculating the medical imaging workflow support data by applying at least one rule of the inference graph to the structured information, and
- providing the medical imaging workflow support data.

Using inference graphs, rules can be implemented using an implication-based, easily understandable format, making the whole process traceable, editable, and explainable even for complex rulesets, such as resulting from clinical standard operating procedure (SOP) guideline data. The inference graph may allow radiologists and technologists to modify preferred workflows through natural language interactions utilizing natural language-(NL)-based finding definitions. The inference graph may allow a rule-driven derivation of protocols based on the structured information items.

Thus, a configurable rule set component may be provided, specifically allowing clinicians and technologists to adjust workflows to their unique requirements, complying with the SOPs defined by individual clinics, even when using natural language only. Furthermore, the proposed solution allows removing the dependency of steadily increasing context sizes to ensure constant learning capabilities, and introducing a novel data-driven learning mechanism allowing to derive SOPs automatically by using the available sets of paired image and indication/report data as provided by the clinical RIS.

The clinical information may be, for example, patient-specific clinical information. In particular, the natural language data may comprise the clinical information in an unstructured format. For example, the natural language data may comprise an unstructured report, the unstructured report comprising the clinical information. The clinical information may comprise, for example, information regarding demographic parameters, in particular gender, weight, height and/or age, physiological parameters, previous findings, previous treatments, a clinical history and/or an anamnesis of a patient. The clinical information may comprise, for example, a referral request and/or an indication and/or a reason for a medical imaging examination.

The large language model may be configured to recognize and/or understand natural language and, in particular, individual items such as words in input containing natural language and transfer the items into a text output. The large language model may be trained and/or machine-learned. The large language model may be provided, for example, by holding the large language model available in a suitable storge accessible by a computing unit executing the method and/or by downloading it by said computing unit.

The large language model may be used for transforming unstructured information into the structured format and/or vice versa. Typical large language models have a specific strength in summarizing and presenting information in the way the user prompts the large language model to do. They have an inherent capability of extracting information from text. The large language model may be configured for natural language processing (NLP), in particular for NLP-based extraction of the structured information from the natural language data.

For example, the structured information may comprise a structured report, the structured report comprising the clinical information in the structured format. The structured format may be, for example, a predefined and/or computer-readable format. The natural language data and/or the structured information may be text-based.

In the structured format, the clinical information can be easily extracted and efficiently communicated in a machine-readable format using predefined, automated communication protocols. Notably, by utilizing a large language model capable of language understanding rather than only parsing, the approach circumvents a variety of major issues arising from the use of merely parsing-based approaches, such as the mentioned inversion problem. In particular, the structured information can be easily parsed for downstream applications, including the integration of additional classical algorithms, or prompting the user to provide the yet missing information, if needed. The structured information may comprise a set of structured information items. These could be represented, for example, by codes from commonly used coding systems (ICD-10, LOINC, CPT, ...) and/or custom, task-specific representations. In particular, the structured information may comprise codes, e.g., ICD-10 or CPT codes, despite the codes not being present in the natural language data.

The medical imaging workflow support data may be, for example, medical imaging decision support data. The medical imaging workflow support data may be calculated, for example, by applying a function for calculating the medical imaging workflow support data to the structured information. The function for calculating the medical imaging workflow support data may be the at least one rule of the inference graph. The function for calculating the medical imaging workflow support data may be trained, in particular machine-learned. For example, the large language model may comprise the function for calculating the medical imaging workflow support data. As an alternative, the function for calculating the medical imaging workflow support data may be rule-based.

A specific advantage of the proposed method results from its strong comprehensibility. As information is presented in a highly structured format, it is easily possible to spot inconsistent information through a human, and thus to reduce novel sources of error as they might result from ontology-based approaches. Further, the reconfigurability of the large language model allows for a simple adaption in case of updated report templates, novel tasks and diseases, and finally the update of previously created structured reports, as reconfigurations correspond to changes in the query, but not changes in the architecture of the large language model.

The large language model may comprise a transformer network. A transformer network is a neural network architecture generally comprising an encoder, a decoder or both an encoder and decoder. In some instances, the encoders and/or decoders are composed of several corresponding encoding layers and decoding layers, respectively. Within each encoding and decoding layer is an attention mechanism. The attention mechanism, sometimes called self-attention, relates data items (such as words or pixels) within a series of data items to other data items within the series. The self-attention mechanism for instance allows the model to examine a word within a sentence and determine the relative importance another word within that sentence has to the word being examined.

The encoder, in particular, may be configured to transform the input (a medical image or text) into a numerical representation. The numerical representation may comprise a vector per input token (e.g., per word). The encoder may be configured to implement an attention mechanism so that each vector of a token is affected by the other tokens in the input. In particular, the encoder may be configured such that the representations resolve the desired output of the transformer network. The decoder, in particular, may be configured to transform an input into a sequence of output tokens. In particular, the decoder may be configured to implement a masked self-attention mechanism so that each vector of a token is affected only by the other tokens to one side of a sequence. Further, the decoder may be auto-regressive meaning in that intermediate results (such as a previously predicted sequence of tokens) are fed back. According to some examples, the output of the encoder is input into the decoder. Further, the transformer network may comprise a classification module or unit configured to map the output of the encoder or decoder to a set of learned outputs such as the text summary.

Training of a transformer model according to some examples may happen in two stages, a pretraining and a fine-tuning stage. In the pretraining stage, a transformer model may be trained on a large corpus of data to learn the underlying semantics of the problem. Such pre-trained transformer models are available for different languages. For certain applications described herein, the fine-tuning may comprise further training the transformer network with medical texts with expert annotated meanings and/or medical ontologies such as RADLEX and/or SNOMED.

With the latter, in particular, the transformer model according to some examples may learn typical relations and synonyms of medical expressions.

The large language model may comprise, for example, a transformer architecture. Transformer networks may use the previously inserted text to find a logical continuation by querying the already provided information, extracting key information, and assigning a value to each. Conducting this process layer by layer, the large language model predicts one token (i.e., word or word part) at a time, with each token being based on the provided information, as well as the world knowledge that is implicitly represented in the network weights.

A query may be received, the query comprising the natural language data and further query data, wherein the structured information is generated by applying the large language model to the natural language data and the further query data. The query may comprise, for example, a user query. The user query may be a free text query the user may input into a user interface, e.g., by typing the user query into an appropriate input filed in the user interface or by voice command. The further query data may comprise template data, the template data being indicative of the structured format. In particular, the large language model may be provided with a dynamically configurable template and queried to transform the natural language data, thereby filling the relevant information into the provided template.

Reference data may be received, the reference data being indicative of rules and/or examples related to the generating the structured information and/or related to the calculating the medical imaging workflow support data, wherein the large language model is tuned based on the reference data, in particular self-tuned and/or fine-tuned based on the reference data.

The large language model may be configured to consider the reference data as an additional source of information, in particular in form of general medical guidelines, site-specific rules and/or preferences, and/or the patient history. The examples may be related to different patients and/or examinations. In particular, the reference data may comprise a corresponding preferred medical imaging decision for different sets of exemplary clinical information. In particular, the further query data may comprise the reference data. In particular, the reference data may be automatically injected as part of the query. Based on the reference data, the large language model may identify patterns and/or reasons why certain recommendations for a new patient and/or exam are desirable, thus allowing, for example, for an on-the-fly learning.

The structured information may comprise a structured information item (SII). The structured information item may comprise the clinical information in the structured format of the at least one input node of the inference graph. The structured information item may be related, for example, to clinical findings and/or the operational coding systems (ICD-10, LOINC, CPT). Structured information items may also be modeled using a description-based natural language variable definition. In an exemplary definition, a structured information item may be specified through natural language only. An exemplary definition would be, e.g., "mention of abnormal Chest XR", "significant artifacts caused by motion", or "tall person (>180cm)". The definition of a structured information item may further comprise possible target values (e.g. true/false), document selectors, and a potential FHIR link.

In particular, a structured information item may comprise one or more of the following properties.

Name: The name of the structured information item. This property may be particularly used in the Inference Graph described below.

Description: A natural-language based description of the structured information item.

Allowed values/value ranges: A definition of allowed values, either in form of a list (e.g. [True, False]), range (e.g. [1...10]) or data type (e.g. bool/int/string/...).

Document selector: Used to select document types from which this finding may be extracted. This is particularly important if structured information items should be considered relevant only if stemming from a specific source, such as a confirmed previous examination report, a laboratory result, etc.

Equivalent Code: An equivalent code in a classical coding scheme, e.g. FHIR, used to link the structured information item to an already existing information system, if any.

Structured information items may be interchangeable with other sources of information, such as FHIR information, or any format of clinically approved findings. The equivalent code therefore serves as a mechanism to link description-based items to specific, e.g. FHIR-based, definitions so that already approved findings may supersede NL-based ones, further improving reliability.

According to a further aspect, the medical imaging workflow support data corresponds to a workflow aspect node of the inference graph. According to a further aspect, the inference graph is based on clinical procedure guideline data and/or on sets of paired patient and workflow data. According to a further aspect, reference data is received, wherein the inference graph is generated by applying a retrieval-augmented generation model to the reference data.

An inference graph may correspond to a set of functions and related parameters and/or may correspond to a graphical representation that illustrates the relationships (edges) between different pieces of information (nodes). It is used to model how conclusions (inferences) can be drawn from given data based on logical connections and/or probabilistic reasoning. Inference graphs help in understanding and visualizing the flow of information and the logical steps taken to reach a conclusion, which is particularly useful in complex NLP tasks such as question answering, information extraction, and reasoning.

The inference graph may be, for example, a trained inference graph model. The inference graph may be, for example, a dynamic inference graph, in particular a dynamically updated inference graph. The clinical procedure guideline data may be, for example, clinical standard operating procedure (SOP) guideline data. The reference data may comprise the clinical procedure guideline data and/or the sets of paired patient and workflow data. The retrieval-augmented generation (RAG) model may first retrieve relevant information from the reference data and/or from the sets of paired patient and workflow data. Then, the retrieval-augmented generation model may use a generative model to generate the inference graph based on the retrieved information. The retrieval-augmented generation model thus combines the strengths of retrieval-based and generation-based models.

Structured information items may be linked in the Inference Graph, in particular to derive a patient-tailored acquisition workflow. The inference graph may comprise a ruleset and/or at least two different types of nodes, denoted, for example, as Information Nodes and Workflow Aspect Nodes. The nodes may correspond to variables.

Information Nodes may be an abstract representation of any kind of information. Information nodes comprise a name, description and - during inference - value. Information Nodes may be, for example, Input Nodes or Intermediate Nodes. In the inference graph, each structured information item is represented as an Input Node. Values of Input Nodes may be populated at the beginning of the inference, while values of Intermediate Nodes may be set through rule application. Information Nodes may not directly correspond to acquisition settings (e.g. keV, mAs, pitch, ...)

Workflow Aspect Nodes may directly correspond to imaging workflow settings and may represent entities such as scans, reconstructions, or post-scanning algorithms. Notably, a Workflow Aspect Node might cover multiple sub-settings, such as a mAs value during scanning, a specific kernel for reconstructions, or a specifically-chosen parameter for an algorithm. In particular, the workflow aspect node of the inference graph may be a target node of the inference graph. These issues may be resolved by bridging the gap between unstructured clinical narratives and the structured data required for efficient workflow automation. By enabling radiologists and technologists to define structured information items (SIIs) only using natural language and integrating them into a rule-driven system, thereby introducing an LLM-powered paradigm shift.

Natural Language Understanding may be used for Seamless Data Extraction. In particular, large language models (LLMs) may be used to autonomously extract relevant clinical information from unstructured reports, translating it into a standardized, machine-readable format represented as structured information items (SIIs) defined only through natural language. This removes the manual mapping bottleneck and ensures data completeness. Furthermore, rules may be created and managed in an intelligent way. In particular, structured information items, expressed in plain language, may form the foundation for creating inference graphs and adaptable rules governing workflow processes in computed tomography imaging. This empowers clinicians and technologists to modify acquisition workflows through natural language interactions, enhancing flexibility and reducing reliance on static rule sets.

An LLM-powered chatbot may offer real-time support, suggesting optimal scan protocols, explaining rule applications, and proactively querying EHR/EMR systems for missing patient context. This fosters data-driven decisions and minimizes the potential for human error. Leveraging the clinical standard operating procedure (SOP) guidelines used for scan workflow configuration and combined with Retrieval Augmented Generation (RAG), large language models (LLMs) can be leveraged to identify the key decision criteria for the scan protocols used at a clinic. This is particularly true for factory protocols, but also ensures adaptability to site-specific SOPs.

According to a further aspect, a representation of the inference graph and/or a representation of a subset of the inference graph is visualized in a user interface, wherein a user input, in particular a manual user input and/or a natural language user input, is received through the user interface, wherein the inference graph is modified based on the user input. The subset of the inference graph may comprise a subgraph of the inference graph and/or a subset of nodes and/or rules of the inference graph. Based on the generated initial rulesets, further refinement using graph-based editing is possible, tremendously reducing the initial effort for workflow and protocol configuration. The graph may be modified using an intuitive graph editing approach. In general, this process is possible in a manual fashion due to the option of visualizing the graph itself. This provides a highly relevant and intuitive fallback solution, in order to accelerate the process of graph modification

Clinical IT systems may store the conducted scans with their respective protocols together and the clinical data of the patient. Therefore, a de-siloing the data may be applied for deriving inference graphs from paired patient and examination data using LLMs through a data mining approach, automatically extracting patient specifics and subsequently analyzing found patterns, allowing to derive general rules that can be represented using the inference graph concept. Notably, the above-proposed data mining approach can similarly be used to analyze continuously changing patterns, as they are typical during the adoption of novel technologies. Therefore, the proposed method allows to slowly adapt with the users as they get more familiar with the novel technologies, and so to further improve clinical workflows over time in a Continuous Learning approach.

According to a further aspect, additional information is queried by an artificial intelligence-based assistance function, in particular in form of a chatbot, from a medical information system and/or from a user. In particular, the inference graph may be modified based on the additional information and/or the medical imaging workflow support data may be calculated based on the additional information. The graph editing interaction may be automated using the artificial intelligence-based assistance function.

In addition to a scan user interface, which would be enriched to display and/or make accessible the derived workflow adaptations, suggestions as well as their basis in terms of rules and source data, an artificial intelligence-based, in particular LLM-based, assistance function, in particular in form of a chatbot, may be used to guide and support the user of the system at any point of the scan and postprocessing workflow. This is of particular importance and benefit considering that making the ideal choices for each patient during imaging and postprocessing grows more complex due to the increasing wealth of possibilities - this holds true especially since the introduction of photon counting CT with "always on" spectral imaging and the possibility to acquire and reconstruct ultra-high resolution (UHR) images.

The indication for the current scan as well as the available structured and unstructured patient history, i.e. the clinical context information which may serve as input for the rule-based workflow automation described above, may also be available to the artificial intelligence-based assistance function. In addition, it may also have access to the rule-set definitions as well as the ideal workflow derived from it based on the clinical context information.

Expert technical and clinical knowledge about the scanner and its capabilities may be provided as well, both by the vendor (as a default, e.g. scanner manuals, whitepapers, ...) or by the user themselves (optionally; custom site-specific know-how and/or SOPs that may not be directly covered or fully explained by the rule-set). This way, the assistance function is well-equipped to reason about and answer any questions regarding the patient, the ideal exam and how to best achieve it using the scanner at hand ("smart user manual").

The technical means with which these documents and related information can be made available to the LLM include direct injection in prompts (system prompt, enriched user prompts) in case the context window and capabilities of the LLM allow for the corresponding amount of text to be processed efficiently and usefully, and/or Retrieval Augmented Generation systems where only relevant parts of the documents are searched and injected dynamically, using, e.g., a vector database that stores pre-computed semantic embeddings of all relevant source documents. The documents may be present in natural (e.g., diagnostic reports) and/or structured language (e.g., derived workflow in a format like JSON or XML) where applicable. LLMs may be fit to flexibly deal with both.

Clinical context information in many cases needs to be retrieved from other systems in the hospital, such as an EHR/EMR (electronic health/medical record) or HIS/RIS/LIS (hospital/radiology/laboratory information system). Integration with these systems on a technical (cf. connectivity) and/or semantic (cf. concept mapping) level allows to use information contained for the purposes described herein. However, the employed LLMs may also assist with the dynamic querying of information. For instance, if a certain piece of information is requested by the user (or identified as a required input of a rule within the given rule set) and is not yet known to be accessible, but may be available within a connected system, the appropriate query (e.g., a FHIR query, i.e. a request for an information resource as defined in the FHIR standard, formulated according to the FHIR communication protocol) may be generated automatically by the LLM and then automatically executed in the background to fetch the corresponding information on demand, if present.

As outlined above, the artificial intelligence-based assistance function may support the user with performing the exam. This may not be limited to answering questions and making suggestions in plain text but also include the possibility to determine and navigate to the source of the provided information and conveniently act on the given suggestions. To this end, the LLM can be instructed to use a prescribed markup language when referring to any input documents or pieces of information as well as when referring to workflow parameters and their values. Specifically, the markup could include a combination of functional descriptors ("jump to document/information", "show parameter value", "set parameter value", "show default/allowed values for parameter", ...) and internal unique identifiers for the documents/information and workflow parameters.

This allows the surrounding system to parse the LLM output for this markup and enable call-up of appropriate functions to provide UI & backend elements and interactions as well as display the markup accordingly in the chat ("Function calling"). Examples could include: Any references to information/documents in the text may be displayed as links that allow the user to hover over it, displaying a pop-over with further information such as the name and timestamp of the source document. Clicking on such a link would switch to a more detailed view of the information in the context of the complete documents with timelines to allow for easy verification, tracing and tracking of information.

Similar links for any mentioned workflow steps would allow, on a click, to switch the view to the part of the scan UI where this parameter is set. In case a specific parameter value is mentioned, clicking the link could immediately set the parameter value accordingly in the backend (potentially guarded by a dialog window to confirm to avoid accidental changes). Using automatic LLM function calling, similar interaction may also be triggered by the LLM directly upon user request. As the LLM has received information about all parameters and potential values, it will be able - given appropriate instructions - to identify if a user request refers to non-existent parameters or disallowed values and respond accordingly. Regardless, the corresponding backend functions may be implemented in a way that invalid requests are ignored even if the LLM were to mistakenly include such a request in its response.

In a similar manner, the artificial intelligence-based assistance function may also allow the user to easily interact not only with the parameters and their values for the current patient, but also the underlying rule set itself. Note that in practice, this typically targets a more specific user role: The rule set, which may reflect the SOPs of the site, would typically be maintained by the lead technologist based on requirements and decisions by radiologists.

As the rule set is represented internally in a formal language describing the graph of dependencies between information items and predefined workflow steps (see above), natural language requests for new or updated rules can be translated by the LLM into the appropriate format and the resulting modification applied to the current rule set representation. The specification of the formal language for defining the rules, i.e. what types of rules can be expressed in what manner, may be given to the LLM as part of the prompt to ensure correct results as well as the ability to identify infeasible requests or ones that are ambiguous and need further clarification with the user.

The scope of changes can seamlessly range from individual targeted modifications to step-by-step creation of complete graphs or subgraphs based on iterative interaction and/or existing documents that describe the desired rules in prose or a structured format such as a spreadsheet. In this sense, the transition to LLM-powered "rule mining" as described above is gradual. As direct editing of big graph structures - even if done visually with a suitable GUI-tool - can be cumbersome even for experts and is beyond the typical expertise and usual way of working of technologists, this solution is expected to significantly reduce the entry barriers for use of the overall system and increase acceptance and convenience.

The artificial intelligence-based assistance function may be used for context awareness and/or acquisition-aware retrieval. The artificial intelligence-based assistance function may be able to comment on the inference graph and/or the derivation of the currently configured workflow, in particular in form of an interactive scanning guide.

Here is an example listing of a user/chatbot ("Assistant") interaction showcasing possibilities for direct interaction with the workflow. Note that the markup is displayed as plain text here for clarity only. In the real user interface such markup may be rendered e.g. in analogy to hyperlinks.

User: Can you outline the most important adaptations you made to the protocol and why you made them?

Assistant: Here are the most important deviations from the defaults of the base protocol <jump_to_param="BaseProtocol">CT Routine Chest (I-)</jump to_param>, which is recommended for <jump to_information_item=CancerStaging>lung cancer staging</jump to_information_item>:
Changed <jump_to_param=ScanProtocol::Scan1::Pitch>pitch</jump_to_param> from 1 to 1.5 because a faster scan can help avoid <jump_to_information_item=MotionArtifacts>motion artifacts</jump_to_information_item> which were observed in a <jump_to_source_document=Report_2024_04_01>prior examination</jump_to_source_document> in April this year.

Turned on <jump to_param=Visualization::RibUnfolding>rib unfolding</jump_to_param> to facilitate detection of metastatic lesions as the patient has a
<jump_to_information_item="HistoryOfCancer">prior history of
cancer</jump_to_information_item>.

User: Alright, can you set the supercalifragiliticity factor to 3?

Assistant: I'm sorry, I don't recognize 'supercalifragiliticity' as a workflow parameter that I am able to modify.

User: Okay, then please at least set the pitch to 100 so the scan goes super fast!

Assistant: Apologies, but the allowed values for pitch within this protocol are
<set_param=ScanProtocol::Scan1::Pitch|0.5>0.5</set_param>,
<set_param=ScanProtocol:: Scan1::Pitch|1>1</set_param>,
<set_param=ScanProtocol::Scan1::Pitch|1.5>1.5</set_param>, and
<set_param=ScanProtocol::Scan1::Pitch|2>2</set_param>. Would you like me to change one of these or change the <jump_to_param=BaseProtocol>base protocol</jump_to_param>?

User: Please change it to 2, then.

Assistant: Understood. <set_param=ScanProtocol::Scan1::Pitch|2>Please click here to confirm the change</set_param>.

User: Also, I need to see the spine too, not just the ribs!

Assistant: Do you wish to generate a
<jump_to_param=Visualization::SpineStraightening>straightened visualization of the spine</jump_to_param>?

User: Yes!

Assistant: Of course. Please click here to
<set_param=Visualization::SpineStraightening|True>confirm</set_param> or reply with "confirm".

User: confirm
Assistant automatically calls function associated with above markup.

In a further aspect, the invention relates to a computer-implemented method for providing medical imaging workflow support data, the method comprising:
- receiving reference data,
- generating the medical imaging workflow support data by applying a retrieval-augmented generation model to the reference data,
- providing the medical imaging workflow support data.

Large language models (LLMs) may be used to create and modify protocol files, in particular in form of XML protocol files, and/or other referenced files, such as the decision tree definitions, based on templates and examples, without the need for a complete definition and specification.

A protocol and example database may be dynamically queried using Retrieval Augmented Generation (RAG) to adapt or synthesize protocols from scratch. Additional documents may be provided, such as, for example, protocol excerpts, scanner manuals, and global or site-specific clinical guidelines that get selected dynamically and optimally with respect to the modifications required.

As LLMs may be highly proficient in working with formal languages and can learn by example without modification of the underlying syntactic structure, by using in-context few-shot learning with example protocol files, they can fill in missing information sensibly based on given instructions and common knowledge derived from their training and example data.

This approach enables the creation and adaptation of scanner-compatible protocol files, i.e. the main protocol XML file as well as any other related files that are required for fully defining protocol, based on a more abstract representation of the desired workflow and scan strategy. Thus, the representation can be kept simple and high-level using formal languages (e.g., JSON), the protocol book (typically digitally available, e.g., as a text or table document), and finally natural language interactions, as was already described with regard to the inference graph.

Using this approach, scanner-specific protocols can be automatically derived by selecting example protocol definitions for the appropriate scanner and/or by providing additional information on the scanner capabilities, such as by including the scanner manual as context. Moreover, the same mechanism can be employed to transform abstract, patient-tailored protocol configurations, as created during exam ordering, protocolling or scheduling, in an EHR and/or a cloud-based environment, into a concrete acquisition protocol for a scanner of choice. This enables a fine-grained scan configuration from a UX-friendly fleet-level viewpoint.

A hybrid approach may be required, in particular if a LLM struggles to reliably perform these tasks for complex protocols given their size and context limitations. In a further aspect, a high-level document pre-segmentation using traditional, domain knowledge-driven methods, may be applied. The overall structure of the scan protocol files and the general semantic hierarchy are typically stable over time and similar for different scanners. The relevant sub-parts are then provided to the LLM for further modification, rendering this approach feasible. Thus, while XML-parsing can be used for meaningful pre-segmentation, the detailed modification and adaption may be handled on a semantical level using the LLM.

The proposed solution may allow to automate this process (automatic protocol maintenance, automatic protocol transfer). Optionally, double-checking the results and making minor targeted corrections could be carried out manually and/or through natural language-based instructions. User interactions on cloud- or EHR-level may initiate the automatic transfer of general or patient-tailored protocols to different scanners or scanners of different versions in an automated fashion. Direct natural-language interactions may result in the creation of, or changes to the scan protocols.

A medical imaging examination workflow step, option and/or parameter may be suggested, selected and/or triggered, in particular automatically suggested, selected and/or triggered, based on the medical imaging workflow support data. The medical imaging workflow support data may be indicative of a value and/or a value change of a scan parameter of a scan protocol for a medical imaging examination of a patient by a medical imaging device. The method for providing medical imaging workflow support data may further comprise: - setting the scan parameter and/or conducting a medical imaging examination based on the scan parameter.

The medical imaging workflow support data may be indicative of a value and/or a value change of a reconstruction parameter of a reconstruction algorithm for reconstructing a medical image based on medical imaging data. The method for providing medical imaging workflow support data may further comprise: - setting the reconstruction parameter and/or reconstructing the medical image based on the medical imaging data. The medical imaging workflow support data may be indicative of a value and/or a value change of an image processing parameter of an image processing algorithm for processing a medical image. The method for providing medical imaging workflow support data may further comprise: - setting the image processing parameter and/or processing the medical image. The image processing algorithm may be configured, for example, for post-processing and/or analysis of the medical image. The post-processing of the medical image may comprise, for example, calculating a representation of an anatomical structure based on the medical image.

The structured information can be used, for example, to enhance and/or automate a scan workflow and/or a post-processing workflow. For this purpose, information from the structured report and the unstructured report as well as the referral request may be automatically structured with respect to the clinical questions relevant to the medical imaging exam to be performed, while including all specific aspects surrounding this concern. Exemplarily, based on the given textual information, the large language model can be used to quickly answer relevant questions such as "May the patient receive contrast agent?", "Is a high-resolution reconstruction recommended for the given indication", "In which organs should an automated search for metastases be performed?", and similar.

The structured information, in particular regarding patient demographics, previous findings or comorbidities, may be used, for example, for selecting and/or triggering the appropriate workflows steps, for choosing appropriate parameters for scanning, reconstruction and post-processing, and as prior information for any machine-learning based image processing algorithm run on the resulting image data set.

In a further aspect, the invention relates to a data processing system for providing medical imaging workflow support data, the data processing system comprising an interface unit and a computing unit,
- wherein the interface unit is configured to receive natural language data, the natural language data comprising clinical information,
- wherein the computing unit is configured to generate structured information by applying a large language model to the natural language data, the structured information comprising the clinical information in a structured format of at least one input node of an inference graph,
- wherein the computing unit is further configured to calculate the medical imaging workflow support data by applying at least one rule of the inference graph to the structured information, and
- wherein the interface unit is further configured to provide the medical imaging workflow support data.

The data processing system may be configured for carrying out the method according to one of the aspects of the invention. The data processing system can comprise, for example, at least one of a cloud-computing system, a distributed computing system, a computer network, a computer, a tablet computer, a smartphone or the like. The data processing system can comprise hardware and/or software. The hardware can be, for example, a processor system, a memory system and combinations thereof. The hardware can be configurable by the software and/or be operable by the software. Calculations for performing an action of a method may be carried out in the processor.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medial image data sets. The interface unit may be further adapted to interface with one or more users of the system, e.g., by displaying the result of the processing by the computing unit to the user (e.g., in a graphical user interface) or by allowing the user to adjust parameters for data processing or visualization. In other words, the interface unit may comprise a user interface.

Data, in particular the natural language data, the query, the further query data, the reference data and the examination request data, can be received, in particular received through the interface unit, for example, by receiving a signal that carries the data and/or by reading the data from a computer memory and/or by a manual user input, for example, through a graphical user interface. Data, in particular the medical imaging workflow support data, can be provided, in particular provided through the interface unit, for example, by transmitting a signal that carries the data and/or by writing the data into a computer memory and/or by displaying the data on a display.

Any of the algorithms, functions and/or models mentioned herein can be based on one or more of the following architectures: deep convolutional neural network, deep belief network, random forest, deep residual learning, deep reinforcement learning, recurrent neural network, Siamese network, generative adversarial network or auto-encoder.

The invention further relates to a medical imaging device comprising the data processing system according to one of the aspects of the invention. The medical imaging device may be configured for carrying out the medical imaging examination of the patient based on the scan protocol for the medical imaging examination. The medical imaging device may be, for example, a computed tomography device, a magnetic-resonance imaging device, an ultrasound imaging device, a PET imaging device, a SPECT imaging device, an X-ray imaging device, a cone beam CT device and/or a combination thereof.

In a further aspect, the invention relates to a computer program product comprising instructions which, when executed by a computer, cause the computer to carry out the method according to one of the aspects of the invention. In a further aspect, the invention relates to a computer-readable storage medium comprising instructions which, when executed by a computer, cause the computer to carry out the method according to one of the aspects of the invention. The realization of the invention by a computer program product and/or a computer-readable storage medium has the advantage that already existing computers can be easily adapted by software updates in order to work as proposed by the invention.

The computer program product can be, for example, a computer program or comprise another element apart from the computer program. This other element can be hardware, for example a memory device, on which the computer program is stored, a hardware key for using the computer program and the like, and/or software, for example, a documentation or a software key for using the computer program. The computer program product may further comprise development material, a runtime system and/or databases or libraries. The computer program product may be distributed among several computer instances. A computer-readable storage medium can be embodied as non-permanent main memory (e.g. random-access memory) or as permanent mass storage (e.g. hard disk, USB stick, SD card, solid state disk).

Utilizing a large language model capable of language understanding, rather than only parsing, poses a significant advantage in comparison to symbolic-ontological methods, that typically struggle with negations, inversions, abbreviations, typos and extra information, either being parsed "hardcoded", and thus very limited, or without being able to handle this information at all. In contrast, language understanding, information extraction and content summarization are key capabilities of typical large language models, allowing to avoid these issues. Specifically, while negations and inversions are an integral part of natural language and thus abundantly represented, world knowledge as well as explicitly given query information can be used to resolve abbreviations and typos. Extra information can be explicitly queried and summarized, such as in form of easily comprehensible bullet points, which themselves may refer to the original report.

As a result, the use of large language models might revolutionize the way in which structured information is created from unstructured natural language data, being a major step toward digital transformation in clinics, improving throughput, accelerating treatment, serving as a double-check for avoiding treatment errors, and finally reducing the workload for the involved parties. With a specific focus on the scan and post-processing workflow automation, such as addressed by the Tech Co-pilot, the advantages are similarly manifold, with the main aspects including time savings through smart scanners, automatically pre-selecting correct workflows and steps, and triggering only relevant and specifically tailored algorithms in order to create relevant (Al-based) image analysis results, standardization, and increased consistency of exams. In summary, this allows for highly reproducible, guidance-based workflow recommendations, and results in a strong potential for error reduction by serving as an automated "second pair of eyes" that is automatically parsing the available text-based sources for any relevant information.

In the context of the present invention, the expression "based on" can in particular be understood as meaning "using, inter alia". In particular, wording according to which a first feature is calculated (or generated, determined etc.) based on a second feature does not preclude the possibility of the first feature being calculated (or generated, determined etc.) based on a third feature. Reference is made to the fact that the described methods and the described systems are merely preferred example embodiments of the invention, and that the invention can be varied by a person skilled in the art, without departing from the scope of the invention as it is specified by the claims.

Features related to the invention or to the technical background of the invention will be illustrated below with reference to the accompanying figures. The illustration in the figures is schematic and highly simplified and not necessarily to scale.
Fig. 1 shows a flow chart for a computer-implemented method for providing medical imaging workflow support data.
Fig. 2 shows a data processing system.
Fig. 3 shows a representation of an inference graph.
Fig. 4 shows a user interface for modifying the inference graph.
Fig. 5 shows a flow chart for a computer-implemented method for protocol modification.

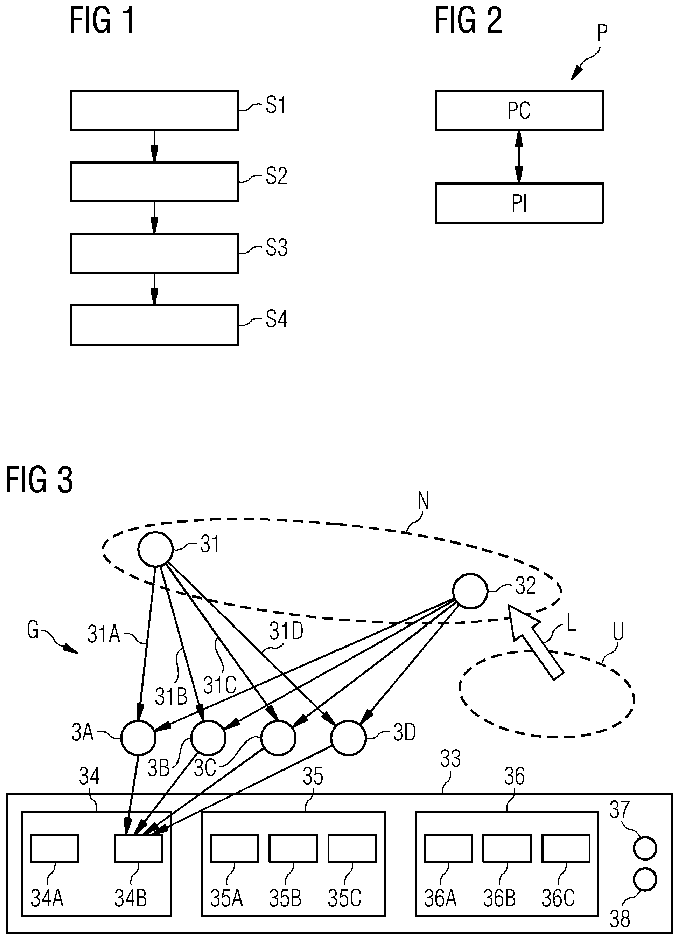

Fig. 1 shows a flow chart for a computer-implemented method for providing medical imaging workflow support data, the method comprising:
- receiving S1 natural language data U, the natural language data U comprising clinical information,
- generating S2 structured information N by applying a large language model L to the natural language data U, the structured information N comprising the clinical information in a structured format of at least one input node 31, 32 of an inference graph G,
- calculating S3 the medical imaging workflow support data by applying at least one rule of the inference graph G to the structured information N, and
- providing S4 the medical imaging workflow support data.

Fig. 2 shows the data processing system P for providing medical imaging workflow support data, the data processing system P comprising an interface unit PI and a computing unit PC,
- wherein the interface unit PI is configured to receive S1 natural language data U, the natural language data U comprising clinical information,
- wherein the computing unit PC is configured to generate S2 structured information N by applying a large language model to the natural language data U, the structured information N comprising the clinical information in a structured format of at least one input node 31, 32 of an inference graph G,
- wherein the computing unit PC is further configured to calculate S3 the medical imaging workflow support data by applying at least one rule of the inference graph G to the structured information N, and
- wherein the interface unit PI is further configured to provide S4 the medical imaging workflow support data.

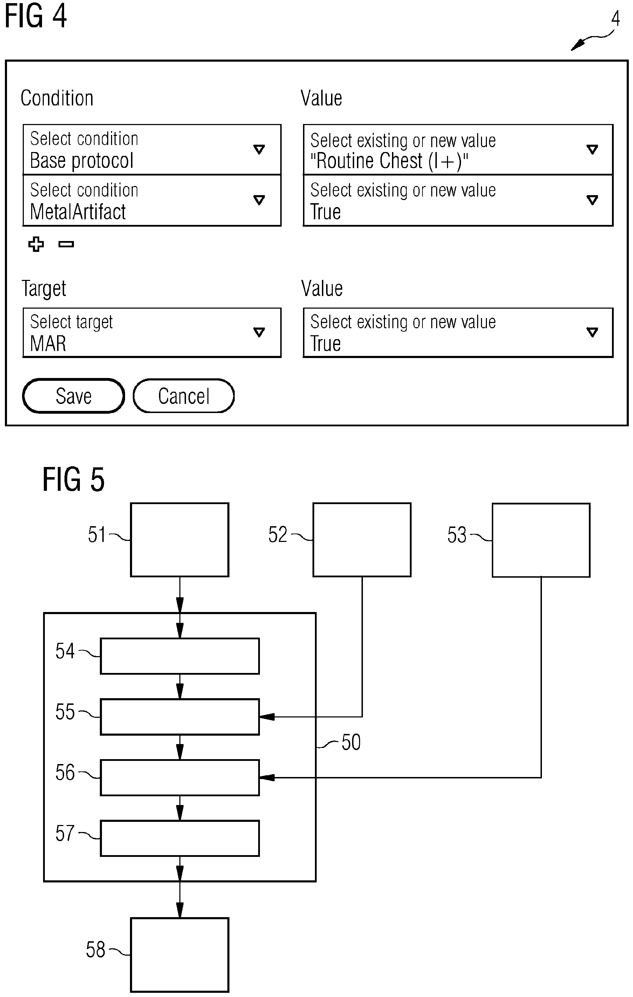

In Fig. 3 and Fig. 4, an exemplary implementation of the manual editing of the inference graph G through the user interface 4 is depicted.

The input node 31 represents a base protocol condition. The input node 32 represents a metal artifact condition. The edges 31A, 31B, 31C and 31D represent Routine Chest (I+), Routine Chest (I-), Nodule Follow-up (I-) and CT PE (I+), respectively, as the potential values of the base protocol. Each of the connections 3A, 3B, 3C and 3D represents a logical conjunction ("AND"). The target nodes within the set 33 are related to reconstruction. The nodes within the subset 34 are related to specific reconstruction algorithms, in particular to iterative reconstruction 34A and to metal artifact reduction 34B, respectively. The nodes within the subset 35 are related to soft thin reconstruction, in particular to a kernel 35A, a slice increment 35B and a slice thickness 35C, respectively. The nodes within the subset 36 are related to dual-energy computed tomography reconstruction, in particular to PBV 36A, VMI 36B and VNC 36C, respectively. Other nodes are related to thick reconstruction 37 and lung thin reconstruction 38.

The medical imaging workflow support data corresponds to a workflow aspect node of the inference graph G. A representation of the inference graph G is visualized in the user interface 4, wherein a user input, in particular a manual user input and/or a natural language user input, is received through the user interface 4, wherein the inference graph G is modified based on the user input. Thus, the inference graph G may be modified using an intuitive graph editing approach based on a visualization of (a subset of) the inference graph G.

Figure 5 shows a flow chart for a computer-implemented method for protocol modification.

The protocol modification function 50 generates the output protocol file 58 based on the input protocol file 51 and the prompt 53, the prompt 53 comprising a description/request (e.g. "The slice thickness of recon three should be halved." or the equivalent code) and/or a link to protocol book data. The reference database 52 comprises additional documents such as protocol excerpts, scanner manuals, and/or global and/or site-specific clinical guidelines that get selected dynamically and optimally by the retrieval-augmented generation model with respect to the modifications required.

The protocol modification function 50 comprises the function 54 for pre-segmentation of the input protocol file 51, the function 55 for context-aware retrieval and retrieval-augmented generation, the function 56 for processing the prompt and modifying the protocol file, and the function 57 for a reassembly to obtain the output protocol file.

## Claims

1. A computer-implemented method for providing medical imaging workflow support data, the method comprising:
- receiving (S1) natural language data (U), the natural language data (U) comprising clinical information,
- generating (S2) structured information (N) by applying a large language model (L) to the natural language data (U), the structured information (N) comprising the clinical information in a structured format of at least one input node (31, 32) of an inference graph (G),
- calculating (S3) the medical imaging workflow support data by applying at least one rule of the inference graph (G) to the structured information (N), and
- providing (S4) the medical imaging workflow support data.

2. The method according to claim 1,
- wherein the medical imaging workflow support data corresponds to a workflow aspect node of the inference graph (G).

3. The method according to claim 1 or 2,
- wherein the inference graph (G) is based on clinical procedure guideline data and/or on sets of paired patient and workflow data.

4. The method according to one of the claims 1 to 3,
- wherein reference data is received,
- wherein the inference graph (G) is generated by applying a retrieval-augmented generation model to the reference data.

5. The method according to one of the claims 1 to 4,
- wherein a representation of the inference graph (G) and/or a representation of a subset of the inference graph (G) is visualized in a user interface (4),
- wherein a user input is received through the user interface (4),
- wherein the inference graph (G) is modified based on the user input.

6. The method according to one of the claims 1 to 5,
- wherein additional information is queried by an artificial intelligence-based assistance function, in particular in form of a chatbot, from a medical information system and/or from a user,
- wherein the inference graph (G) is modified based on the additional information and/or wherein the medical imaging workflow support data is calculated based on the additional information.

7. A computer-implemented method for providing medical imaging workflow support data, the method comprising:
- receiving reference data,
- generating the medical imaging workflow support data by applying a retrieval-augmented generation model to the reference data,
- providing the medical imaging workflow support data.

8. The method according to one of the claims 1 to 7,
- wherein a medical imaging examination workflow step, option and/or parameter is suggested, selected and/or triggered based on the medical imaging workflow support data.

9. The method according to one of the claims 1 to 8,
- the medical imaging workflow support data being indicative of a value and/or a value change of a scan parameter of a scan protocol for a medical imaging examination of a patient by a medical imaging device.

10. The method according to one of the claims 1 to 9,
- the medical imaging workflow support data being indicative of a value and/or a value change of a reconstruction parameter of a reconstruction algorithm for reconstructing a medical image based on medical imaging data.

11. The method according to one of the claims 1 to 10,
- the medical imaging workflow support data being indicative of a value and/or a value change of an image processing parameter of an image processing algorithm for processing a medical image.

12. A data processing system (P) for providing medical imaging workflow support data, the data processing system (P) comprising an interface unit (PI) and a computing unit (PC),
- wherein the interface unit (PI) is configured to receive (S1) natural language data (U), the natural language data (U) comprising clinical information,
- wherein the computing unit (PC) is configured to generate (S2) structured information (N) by applying a large language model to the natural language data (U), the structured information (N) comprising the clinical information in a structured format of at least one input node (31, 32) of an inference graph (G),
- wherein the computing unit (PC) is further configured to calculate (S3) the medical imaging workflow support data by applying at least one rule of the inference graph (G) to the structured information (N), and
- wherein the interface unit (PI) is further configured to provide (S4) the medical imaging workflow support data.

13. A medical imaging device comprising the data processing system (P) of claim 12.

14. A computer program product comprising instructions which, when executed by a computer, cause the computer to carry out the method of one of claims 1 to 11.

15. A computer-readable storage medium comprising instructions which, when executed by a computer, cause the computer to carry out the method of one of claims 1 to 11.
